# EUROPÄISCHE PATENTANMELDUNG

(11) **EP 4 785 885 A1**
(43) Veröffentlichungstag der Anmeldung: **05.08.2026**
(21) Anmeldenummer: 25155481.2
(22) Anmeldetag: 03.02.2025
(51) Int. Cl.: A61B 17/72

(54) **OSTEOSYNTHESE-MARKNAGELANORDNUNG**

(71) Anmelder: Medizinischer Sachverständiger Roncevic GbR, 48308 Senden (DE)
(72) Erfinder: Roncevic, Bozidar, 48329 Havixbeck (DE)
(74) Vertreter: Kalkoff & Partner Patentanwälte mbB

(57) **Zusammenfassung**

Die Erfindung betrifft eine Osteosynthese-Marknagelanordnung (1) zur Osteosynthese von Knochenteilen (2, 3) eines frakturierten Röhrenknochens (4).

## Beschreibung

Die Erfindung betrifft eine Osteosynthese-Marknagelanordnung zur Osteosynthese von Knochenteilen eines frakturierten Röhrenknochens.

Frakturierte Röhrenknochen können herkömmlich mit einem Marknagel versorgt werden, der hierzu in die Markräume von miteinander zu verbindenden Knochenteile eingefügt wird, um eine biomechanisch optimale und belastungsstabile (koaxiale) Versorgung solcher Röhrenknochenfrakturen auszubilden. Über quer zur Längserstreckung des Marknagels verlaufende Verriegelungsschrauben ist eine Längen- und Torsionsstabilisierung erzielbar, indem die Verriegelungsschrauben durch das jeweilige Knochenteil und durch den Marknagel geführt werden.

Marknägel sind üblicherweise hohl. Sie können beispielsweise eine Breite von 1 cm und eine Wanddicke von 1 mm aufweisen.

Zur Reposition der Knochenfragmente wird üblicherweise ein Führungsdraht in die Markräume beider Knochenteile eingeschoben. Die Marknägel weisen axial verlaufende, durchgängige, zentrierte Kanäle auf. Ein Nagel wird auf den Führungsdraht aufgeschoben bzw. aufgefädelt. Anschließend wird der Marknagel über den Führungsdraht gleitend in die Markräume der Knochenfragmente eingeschoben. Anschließend wird der Draht wieder entfernt. Die zentrierten Kanäle führen dazu, dass der Marknagel zwangsläufig zentriert im Markraum positioniert werden kann.

Einige Marknägel eignen sich für eine Dynamisierung, d.h. eine Bewegung der Knochenteile relativ zueinander. Allerdings ist es hier nachteilig, dass für eine Dynamisierung manche Schrauben in einer weiteren Operation entfernt werden müssen. Hierbei ist der richtige Zeitpunkt abzuschätzen: Auf der einen Seite darf die Entfernung nicht zu früh erfolgen, da die sonst verbliebenen Schrauben zu brechen drohen. Auf der anderen Seite sollte die Entfernung nicht zu spät erfolgen, da der Marknagel andernfalls bereits verwachsen ist; eine Dynamisierung ist nicht mehr möglich.

Die Dynamisierung erfolgt oftmals über ein Langloch im Marknagel, wobei die Dynamisierung nicht kontrollierbar, d.h. nicht einstellbar ist. Weiterhin verliert die Konstruktion durch das Entfernen der Schrauben an Stabilität mit der Folge, dass der Patient den frakturierten Knochen nicht mehr belasten darf. Ebenso wenig ist es möglich, die Dynamisierung intraoperativ, d.h. während der Operation, in der der Nagel gesetzt wird, vorzunehmen. Tritt bei der Fixierung eines Marknagels ein Spalt zwischen den miteinander zu verbindenden Knochenteilen auf, ist dies oftmals ein Hauptgrund für Komplikationen. Weiterhin erfolgt die Dynamisierung herkömmlich lediglich unilateral, d.h. lediglich ein Knochenteil ist beweglich.

WO 2006 / 040 612 A1 betrifft einen einteiligen Marknagel. DE 199 12 696 A1 betrifft einen in seiner Länge einstellbaren Marknagel.

Es ist eine Aufgabe der Erfindung, eine Osteosynthese-Marknagelanordnung zur Osteosynthese von Knochenteilen eines frakturierten Röhrenknochens bereitzustellen, mit der eine Dynamisierung der Fraktur möglich ist. Hierbei soll die Höhe / Länge der Dynamisierung einstellbar sein. Die Dynamisierung soll intraoperativ möglich sein. Zudem soll eine bilaterale Dynamisierung gegeben sein. Ein Entfernen von Schrauben zur Dynamisierung soll nicht erforderlich sein.

Diese Aufgabe wird durch den Gegenstand des Patentanspruchs 1 gelöst. Vorteilhafte Ausgestaltungen sind in den Unteransprüchen, der nachfolgenden Beschreibung und den Figuren wiedergegeben, wobei diese Ausgestaltungen jeweils für sich genommen oder Kombination von wenigstens zwei dieser Ausgestaltungen miteinander einen vorteilhaften und/oder weiterbildenden Aspekt der Erfindung darstellen können.

Eine erfindungsgemäße Osteosynthese-Marknagelanordnung zur Osteosynthese von Knochenteilen eines frakturierten Röhrenknochens weist wenigstens Marknagel mit variierbarer axialer Länge auf, wobei der Marknagel umfasst
- wenigstens ein erstes Nagelelement in Form einer Aufnahmehülse, die zumindest teilweise in einen Markraum eines ersten Knochenteils des frakturierten Röhrenknochens einfügbar ist, wobei ein Hülsenabschnitt des ersten Nagelelements über wenigstens eine Verriegelungsschraube, bevorzugt zwei bis fünf Verriegelungsschrauben, an dem ersten Knochenteil fixierbar ist,
- wenigstens ein teilweise axial in das erste Nagelelement eingreifendes zweites Nagelelement in Form eines Nagels, das einen außerhalb des ersten Nagelelements angeordneten, in einen Markraum des zweiten Knochenteils einfügbaren und an dem zweiten Knochenteil fixierbaren Fixierabschnitt aufweist, über das das Nagelelement mit wenigstens einer Verriegelungsschraube, bevorzugt zwei bis fünf Verriegelungsschrauben, an dem zweiten Knochenteil fixierbar ist, und
- wenigstens eine Begrenzungseinrichtung zum Begrenzen einer axialen Beweglichkeit des zweiten Nagelelements relativ zu dem ersten Nagelelement in Richtung eines dem Fixierabschnitt abgewandten Ende des ersten Nagelelements,
wobei an einem in dem Hülsenabschnitt befindlichen Nagelabschnitt des zweiten Nagelelements wenigstens eine quer zu einer Längserstreckung des zweiten Nagelelements verlaufende Durchbrechung in Form eines sich axial erstreckenden Langlochs ausgebildet ist, durch die die Verriegelungsschraube hindurchgeführt ist.

Im Folgenden wird das zweite Nagelteil in Form eines Nagels alternativ als Nagelteil bezeichnet.

Mit der erfindungsgemäßen Osteosynthese-Marknagelanordnung kann durch eine Betätigung der Begrenzungseinrichtung eine Dynamisierung erfolgen, ohne dass die zur Fixierung des Marknagels eingesetzten Verriegelungsschrauben entfernt werden müssen. Damit lässt sich eine weitere Operation zur Entfernung der Verriegelungsschrauben vermeiden. Zudem führt dies zu einer höheren Stabilisierung und einer besseren Belastbarkeit des Knochens durch den Patienten. Darüber hinaus kann mit der erfindungsgemäßen Osteosynthese-Marknagelanordnung die Dynamisierung intraoperativ vorgenommen werden. Letzteres ist beispielsweise dann erforderlich, wenn während der Operation der Marknagel mit den Verriegelungsschrauben fixiert ist, dann aber doch ein Spalt zwischen den Knochenteilen der Fraktur auftritt. Darüber hinaus ist es mit der erfindungsgemäßen Osteosynthese-Marknagelanordnung möglich, unilaterale proximale, unilaterale distale sowie bilaterale Dynamisierungen eines frakturierten Knochens vorzunehmen. Der erfindungsgemäße Marknagel kann beispielsweise derart ausgebildet sein, dass die Dynamisierung bzw. die Verkürzbarkeit des erfindungsgemäßen Marknagels beispielsweise auf maximal 15 mm, insbesondere 10 mm, eingeschränkt ist, da in der Praxis eine höhere Dynamisierung kaum erforderlich ist. Allerdings soll die vorliegende Erfindung nicht zwingend auf eine solche maximale Dynamisierung eingeschränkt sein.

Soll ein frisch frakturierter Röhrenknochen mittels der erfindungsgemäßen Osteosynthese-Marknagelanordnung versorgt werden, wird der Röhrenknochen zunächst reponiert (temporär fixiert). Der Operateur verfolgt das Ziel, eine Wiederherstellung des Knochens zu erreichen, wie er vor dem Bruch war. Zur temporären Fixation werden die Knochenteile zueinander geführt und mit einem dazu geeigneten Instrumentarium, das dem Fachmann bekannt ist, fixiert. Anschließend wird der Marknagel in den Markraum der Knochenteile des frakturierten Röhrenknochens eingefügt. Das Einfügen kann anterograd oder retrograd erfolgen. Das Einfügen, auch Nagelinsertion genannt, kann mittels eines Drahts erfolgen. Für diese Anwendung weißt die Anordnung einen axial verlaufenden, durchgängigen, zentrierten Kanal aufweisen, um einen Draht zur Nagelinsertion aufzunehmen. Diese Art der Insertion ist dem Fachmann bekannt. Abschließend wird der Marknagel mittels Verriegelungsschrauben an den Knochenteilen fixiert.

Üblicherweise wird intraoperativ eine Kontroll-Röntgenaufnahme durchgeführt. Dabei ist es möglich, dass trotz des Reponierens der Knochenteile des frakturierten Röhrenknochens ein Spalt zwischen den Knochenteilen vorhanden ist. Dieser Spalt kann durch die erfindungsgemäße Osteosynthese-Marknagelanordnung intraoperativ beseitigt werden, indem die Begrenzungseinrichtung derart betätigt wird, dass die axiale Länge des Marknagels mittels der Begrenzungseinrichtung variiert wird. Hierbei kann die axiale Beweglichkeit der Aufnahmehülse und des Nagelteils relativ zueinander von 0 mm auf einen Wert kleiner oder gleich 15 mm, vorzugsweise kleiner oder gleich 10 mm, erhöht werden, wodurch sich bei einer Belastung des Marknagels die axiale des Marknagels verringert wird, also der Marknagel axial verkürzt wird.

Der Marknagel ist axial teleskopierbar ausgeführt, indem eine Variation der axialen Länge des Marknagels durch eine axiale Verschiebung der Aufnahmehülse und des Nagelteils relativ zueinander vorgenommen wird. Die Dynamisierung wird insbesondere durch die sich axial erstreckenden Langlöcher ermöglicht, die sich in einem Nagelabschnitt des zweiten Nagelelements befinden.

Die Aufnahmehülse und/oder das Nagelteil können teilweise oder vollständig aus einem Metall oder einer Metalllegierung, beispielsweise einer Titanlegierung, gebildet sein. Die Aufnahmehülse und/oder das Nagelteil können unterschiedliche Längen aufweisen. Hierdurch können verschiedenen Knochenarten, Knochenlängen und die Position der Fraktur bei der Wahl eines geeigneten Marknagels berücksichtigt werden. Eine Wanddicke der Aufnahmehülse und/oder des Nagelteils kann beispielsweise 1 mm betragen. Das zweite Nagelelement ist üblicherweise hohl, um eine gewisse Elastizität aufzuweisen, und kann eine ähnliche Wanddicke aufweisen. Ein radialer Abstand zwischen der Aufnahmehülse und dem Nagelteil kann beispielsweise derart dimensioniert sein, dass die Aufnahmehülse frei auf dem Nagelteil gleiten kann. Ein radialer Abstand zwischen dem zu behandelnden Knochen und dem Marknagel kann derart dimensioniert sein, dass der Marknagel im Rahmen der intramedullären Osteosynthese in den Markraum des Knochens "eingeschoben" werden kann und dass bei der Dynamisierung beide Knochenteile bzw. Frakturfragmente noch axial auf dem Marknagel gleiten können.

Dadurch, dass die Aufnahmehülse teilweise in einen Markraum eines ersten Knochenteils des frakturierten Röhrenknochens und teilweise in einen Markraum des zweiten Knochenteils des frakturierten Röhrenknochens einfügbar ist, kann der frakturierte Röhrenknochen im Bereich der Fraktur sehr gut mittels der Aufnahmehülse verstärkt bzw. stabilisiert werden.

Die Begrenzungseinrichtung kann wenigstens einen Anschlag aufweisen, der die erste axiale Endstellung der axialen Beweglichkeit der Aufnahmehülse und des Nagelteils relativ zueinander definiert, wenn der Marknagel seine maximale axiale Länge aufweist. Vorzugsweise wird der Marknagel mit seiner maximalen axialen Länge in den frakturierten Röhrenknochen eingebracht, wobei hierbei mittels der Begrenzungseinrichtung die axiale Beweglichkeit der Aufnahmehülse und des Nagelteils relativ zueinander vorzugsweise auf 0 mm begrenzt ist. Anschließend kann die Begrenzungseinrichtung beispielsweise derart betätigt werden, dass mittels der Begrenzungseinrichtung die axiale Beweglichkeit individuell auf einen Wert in dem Bereich von >0 mm bis maximal 15 mm, vorzugsweise bis maximal 10 mm, eingestellt bzw. festgelegt wird. Dadurch sind also die Aufnahmehülse und das Nagelteil axial in einem Bereich von >0 mm bis maximal 15 mm, vorzugsweise bis maximal 10 mm, relativ zueinander bewegbar, wodurch eine gewünschte Dynamisierung bewirkt wird.

Die erfindungsgemäße Osteosynthese-Marknagelanordnung dient insbesondere zur Osteosynthese von Knochenteilen eines frakturierten Röhrenknochens, insbesondere eines Oberarmknochens, eines Unterarmknochens (Elle, Speiche), eines Oberschenkelknochens, eines Schienbeinknochens, eines Wadenbeinknochens oder dergleichen.

Gemäß einer vorteilhaften Ausgestaltung weist die Begrenzungseinrichtung wenigstens ein an einer Innenmantelfläche der Aufnahmehülse angeordnetes Innengewinde und wenigstens eine in das Innengewinde einschraubbare Madenschraube auf, wobei die zweite Endstellung der axialen Beweglichkeit mittels der Madenschraube individuell festlegbar ist. Die Dynamisierung kann dadurch mehrfach und schrittweise geändert werden. Eine axiale Höhe des Innengewindes kann beispielsweise wenigstens dem Doppelten einer axialen Höhe der Madenschraube entsprechen, während die axiale Höhe der Madenschraube beispielsweise der maximalen Beweglichkeit der Aufnahmehülse und des Nagelteils relativ zueinander entsprechen kann. Die Madenschraube bildet einen axialen Anschlag für das Nagelteil und begrenzt somit die Beweglichkeit des Nagelteils in einer axialen Richtung relativ zu der Aufnahmehülse. Die Madenschraube kann an ihrer dem Nagelteil abgewandten Seite wenigstens eine Mitnehmerkontur aufweisen, mit der ein Werkzeug formschlüssig verbunden werden kann, um die Madenschraube betätigen zu können. Die Mitnehmerkontur kann beispielsweise als Inbus-, Schlitz oder Kreuzschlitzkontur oder dergleichen ausgebildet sein.

Gemäß einer weiteren vorteilhaften Ausgestaltung ist ein in den Markraum des ersten Knochenteils einfügbarer Hülsenabschnitt der Aufnahmehülse über wenigstens eine Verriegelungsschraube an dem ersten Knochenteil fixierbar, wobei an einem in dem Hülsenabschnitt befindlichen Nagelabschnitt des Nagelteils wenigstens eine quer zu einer Längserstreckung des Nagelteils verlaufende Durchbrechung in Form eines sich axial erstreckenden Langlochs ausgebildet ist, durch die die Verriegelungsschraube hindurchgeführt ist, und wobei ein der in das Innengewinde eingeschraubten Madenschraube zugewandt angeordnetes Ende der Durchbrechung die erste axiale Endstellung der axialen Beweglichkeit definiert. Hierdurch ist das Nagelteil nach einer Betätigung der Madenschraube, durch die die Madenschraube von dem Nagelteil wegbewegt wird, trotz der Fixierung mittels der Verriegelungsschraube axial relativ zu der Aufnahmehülse verlagerbar angeordnet, und zwar derart, dass diese axiale Verlagerbarkeit bzw. Beweglichkeit einerseits durch die Madenschraube und andererseits durch das der in das Innengewinde eingeschraubten Madenschraube zugewandt angeordnete Ende der Durchbrechung begrenzt ist. An dem Hülsenabschnitt ist hierbei wenigstens eine quer zu einer Längsmittelachse des Hülsenabschnitts verlaufende Fixierungsbohrung ausgebildet, durch die die Verriegelungsschraube formschlüssig hindurchgeführt ist. Durch einen Formschluss zwischen der Durchbrechung an dem Nagelteil und der durch diese Durchbrechung hindurchgeführten Verriegelungsschraube, der dadurch ausgebildet sein kann, dass eine Breite der Durchbrechung einem Außendurchmesser der durch diese Durchbrechung hindurchgeführten Verriegelungsschraube entspricht, kann das Nagelteil gegen eine Verdrehung um seine Längsmittelachse relativ zu dem ersten Knochenteil und zu der Aufnahmehülse gesichert werden. Auch die Aufnahmehülse wird mittels der Verriegelungsschraube und eines Formschlusses zwischen der Fixierungsbohrung an dem Hülsenabschnitt und der durch diese Durchbrechung hindurchgeführten Verriegelungsschraube gegen eine Verdrehung um ihre Längsmittelachse relativ zu dem ersten Knochenteil gesichert. Die Fixierungsbohrung ist vorzugsweise als Durchgangsbohrung mit kreisrunder Querschnittsfläche ausgebildet. Da die Verriegelungsschraube in Längsrichtung des Nagelteils bzw. des Langlochs etwas Spiel relativ zu dem Nagelteil hat, kann die Heilung des Röhrenknochens dynamisiert werden.

Gemäß einer weiteren vorteilhaften Ausgestaltung ist der Hülsenabschnitt über wenigstens drei in einer axialen Reihe beabstandet zueinander angeordnete Verriegelungsschrauben an dem ersten Knochenteil fixierbar, wobei an dem Nagelabschnitt drei quer zu der Längserstreckung des Nagelteils verlaufende, in einer axialen Reihe beabstandet zueinander angeordnete Durchbrechungen in Form von sich axial erstreckenden Langlöchern ausgebildet sind, durch die die Verriegelungsschrauben hindurchgeführt sind. Dass die Durchbrechungen an dem Nagelabschnitt bzw. Nagelteil in einer axialen Reihe beabstandet zueinander angeordnet sind, bedeutet, dass diese Durchbrechungen bezüglich einer Längsmittelachse des Nagelteils in einer axialen Reihe beabstandet zueinander angeordnet sind.

Gemäß einer weiteren vorteilhaften Ausgestaltung weist die Osteosynthese-Marknagelanordnung wenigstens eine teilweise in das Innengewinde einschraubbare Verschlusskappe auf, mit der ein auf einer dem Nagelteil abgewandten Seite der in das Innengewinde eingeschraubten Madenschraube angeordnetes axiales Ende der Aufnahmehülse verschließbar ist. Hierdurch kann beispielsweise verhindert werden, dass Knochenmark beim Einfügen der Aufnahmehülse in den Markraum des ersten Knochenteils des Röhrenknochens in die Aufnahmehülse eindringt. Zudem kann durch die Anordnung der Verschlusskappe an dem axialen Ende der Aufnahmehülse verhindert werden, dass Gewebe in den Nagel einwächst, was unbedingt vermieden werden sollte.

Gemäß einer weiteren vorteilhaften Ausgestaltung entspricht ein Außendurchmesser der Fixierabschnitts einem Außendurchmesser der Aufnahmehülse.

Gemäß einer weiteren vorteilhaften Ausgestaltung ist der Fixierabschnitt über wenigstens zwei in einer axialen Reihe beabstandet zueinander angeordnete Verriegelungsschrauben an dem zweiten Knochenteil fixierbar, wobei an dem Fixierabschnitt wenigstens zwei quer zu einer Längserstreckung des Nagelteils verlaufende Bohrungen ausgebildet sind, durch die jeweils eine der Verriegelungsschrauben hindurchführbar ist. Dass die Bohrungen an dem Fixierabschnitt des Nagelteils in einer axialen Reihe beabstandet zueinander angeordnet sind, bedeutet, dass diese Bohrungen bezüglich einer Längsmittelachse des Nagelteils und/oder der Aufnahmehülse in einer axialen Reihe beabstandet zueinander angeordnet sind. Jede Bohrung des Fixierabschnitts ist vorzugsweise als Durchgangsbohrung mit kreisrunder Querschnittsfläche ausgebildet. Der Fixierabschnitt kann beispielsweise über drei in einer axialen Reihe beabstandet zueinander angeordnete Verriegelungsschrauben an dem zweiten Knochenteil fixierbar sein, wobei hierzu an dem Fixierabschnitt drei quer zu der Längserstreckung des Nagelteils verlaufende Bohrungen ausgebildet sein können, durch die jeweils eine der Verriegelungsschrauben hindurchführbar ist.

In einer alternativen Ausführungsform der Erfindung umfasst die Marknagelanordnung ein drittes Nagelelement in Form einer Aufnahmehülse, die das zweite Nagelelement teilweise umschließt. Das dritte Nagelelement ist zumindest teilweise in den Markraum des zweiten Knochenteils einfügbar. Das dritte Nagelelement weist einen Hülsenabschnitt auf, die über Durchbrechungen verfügt, durch die die Verriegelungsschrauben durch das zweite Nagelelement und den an dem zweiten Knochenteil fixierbaren Fixierabschnitt des zweiten Nagelelements durchführbar sind, wodurch drittes Nagelelement und zweites Nagelelement am zweiten Knochenteil fixierbar sind. Das dritte Nagelelement kann mit dem zweiten Nagelelement verschweißt sein.

Gemäß einer weiteren vorteilhaften Ausgestaltung sind bzw. ist die Aufnahmehülse und/oder das Nagelteil zumindest teilweise gekrümmt ausgebildet. Dies kann insbesondere bei der Behandlung eines gekrümmt ausgebildeten, frakturierten Röhrenknochens sinnvoll sein, beispielsweise zur Behandlung eines Schienbeinknochens, wobei die Krümmung beispielsweise in einem Bereich von 2° bis 9° liegen kann.

Gemäß einer weiteren vorteilhaften Ausgestaltung weist die Begrenzungseinrichtung wenigstens ein elektromotorisches Stellmittel auf, mit dem eine Position der in das Innengewinde eingeschraubten Madenschraube relativ zu der Aufnahmehülse variierbar ist. Mittels des elektromotorischen Stellmittels, dass vorzugsweise kabellos von außen ansteuerbar ist, kann die Verlagerung des Nagelteils relativ zu der Aufnahmehülse sehr kontrolliert über eine Betätigung der Madenschraube freigegeben werden, ohne dass hierzu eine anderweitige Behandlung eines Pateienten erforderlich wäre. Gemäß einer weiteren vorteilhaften Ausgestaltung ist die Begrenzungseinrichtung ausschließlich mechanisch ausgebildet. Die Begrenzungseinrichtung kann dabei beispielsweise mittels eines Werkzeugs betätigt werden.

In einer weiteren vorteilhaften Ausführungsform umfasst die Marknagelanordnung wenigstens eine weitere Begrenzungseinrichtung zum verliersicheren Begrenzen einer axialen Beweglichkeit des zweiten Nagelelements relativ zu dem ersten Nagelelement in Richtung eines dem Fixierabschnitt des abgewandten Endes des ersten Nagelelements weg.

Im Folgenden wird die Erfindung unter Bezugnahme auf die beigefügten Figuren anhand einer bevorzugten Ausführungsform beispielhaft erläutert, wobei die nachfolgend genannten Merkmale sowohl jeweils für sich genommen als auch in unterschiedlicher Kombination miteinander einen vorteilhaften und/oder weiterbildenden Aspekt der Erfindung darstellen können. Es zeigt:
- Fig. 1: einen schematischen Längsschnitt eines Ausführungsbeispiels für eine erfindungsgemäße Marknagelanordnung;
- Fig. 2: einen weiteren schematischen Längsschnitt der in Fig. 1 gezeigten Marknagelanordnung;
- Fig. 3: einen schematischen Längsschnitt von Details der in Fig. 1 gezeigten Marknagelanordnung in einem Ausgangszustand;
- Fig. 4: einen schematischen Längsschnitt von Details der in Fig. 1 gezeigten Marknagelanordnung in einem Dynamisierungszustand;
- Fig. 5: einen schematischen Längsschnitt von Details der in Fig. 1 gezeigten Marknagelanordnung in einem weiteren Dynamisierungszustand; und
- Fig. 6: einen schematischen Längsschnitt von Details der in Fig. 1 gezeigten Marknagelanordnung in einem weiteren Dynamisierungszustand.

In den Figuren sind gleiche bzw. funktionsgleiche Bauteile mit denselben Bezugszeichen versehen. Eine wiederholte Beschreibung solcher Bauteile kann weggelassen sein.

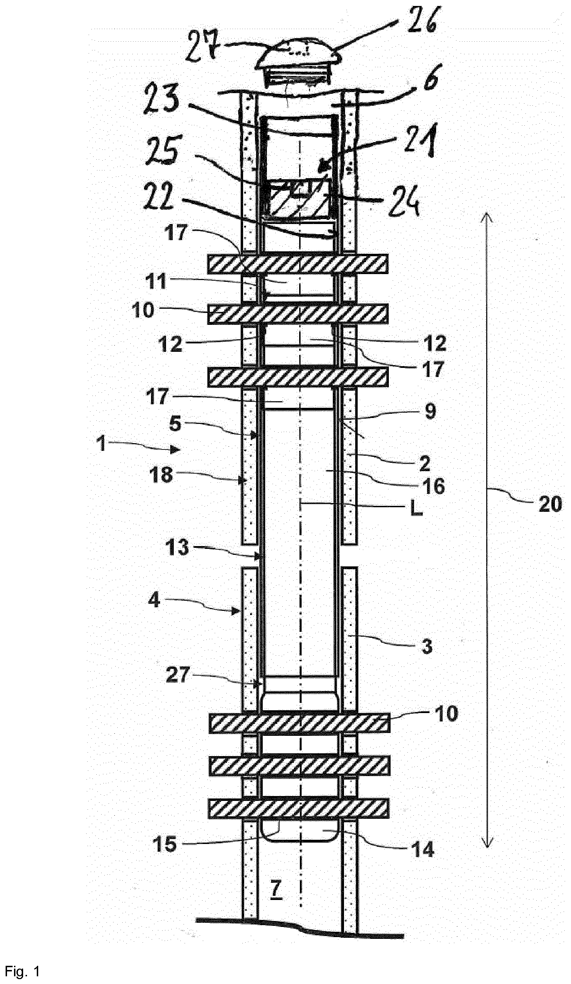

Fig. 1 zeigt einen schematischen Längsschnitt eines Ausführungsbeispiels für eine erfindungsgemäße Osteosynthese-Marknagelanordnung 1 zur Osteosynthese von Knochenteilen 2 und 3 eines frakturierten Röhrenknochens 4.

Der Marknagel 1 kann beispielsweise derart ausgebildet sein, dass die Dynamisierung bzw. die Verkürzbarkeit des Marknagels 1 beispielsweise auf maximal 10 mm eingeschränkt ist, da in der Praxis eine höhere Dynamisierung kaum erforderlich ist. Allerdings soll die vorliegende Erfindung nicht zwingend auf eine solche maximale Dynamisierung eingeschränkt sein.

Die Osteosynthese-Marknagelanordnung 1 weist einen Marknagel 18 mit variierbarer axialer Länge 20 auf.

Der Marknagel 18 weist eine beispielsweise proximal anordbare, Aufnahmehülse 5 auf, die teilweise in einen Markraum 6 eines ersten Knochenteils 2 des frakturierten Röhrenknochens 4 und teilweise in einen Markraum 7 eines zweiten Knochenteils 3 des frakturierten Röhrenknochens 4 eingefügt ist.. Die Aufnahmehülse 5 kann eine Wanddicke von 1 mm aufweisen.

Ein in den Markraum 6 des ersten Knochenteil 2 eingefügter Hülsenabschnitt 9 der Aufnahmehülse 5 ist über drei in einer axialen Reihe beabstandet zueinander angeordnete Verriegelungsschrauben 10 an dem ersten Knochenteil 2 fixiert. Hierzu sind an dem Hülsenabschnitt 9 drei quer zu einer Längserstreckung L der Aufnahmehülse 5 verlaufende, in einer axialen Reihe zueinander angeordneten Durchbrechungen 11 ausgebildet. Jede Durchbrechung 11 weist zwei bezüglich der Längsmittelachse L der Aufnahmehülse 5 diametral einander gegenüberliegende Durchbrechungsöffnungen 12 auf. Die jeweilige Verriegelungsschraube 10 ist durch die jeweilige Durchbrechung 11 hindurchgeführt. Jede Durchbrechung 11 ist als Durchgangsbohrung mit kreisrunder Querschnittsfläche ausgebildet.

Zudem weist der Marknagel 18 ein teilweise axial in die Aufnahmehülse 5 eingreifendes, beispielsweise distal anordbares, Nagelteil 13 auf, das einen außerhalb der Aufnahmehülse 5 angeordneten, in den Markraum 7 des zweiten Knochenteils 3 eingefügten und an dem zweiten Knochenteil 3 fixierten Fixierabschnitt 14 aufweist. Ein Außendurchmesser der Fixierabschnitts 14 entspricht einem Außendurchmesser der Aufnahmehülse 5. Eine Wanddicke des Nagelteils 13 kann beispielsweise 1 mm betragen. Ein axialer Abstand zwischen dem Fixierabschnitt 14 und der Aufnahmehülse 5 beträgt in dem gezeigten Ausführungsbeispiel 10 mm. Eine Differenz zwischen dem Außendurchmesser der Aufnahmehülse 5 und dem Außendurchmesser des Nagelabschnitts 16 des Nagelteils 13 beträgt in dem gezeigten Ausführungsbeispiel etwa 1,5 mm.

Der Marknagel 18 weist zudem eine Begrenzungseinrichtung 21 zum Begrenzen einer axialen Beweglichkeit der Aufnahmehülse 5 und des Nagelteils 13 relativ zueinander auf. Die Begrenzungseinrichtung 21 ist derart ausgebildet und angeordnet, dass die axiale Beweglichkeit der Aufnahmehülse 5 und des Nagelteils 13 relativ zueinander individuell in einem Bereich von 0 mm bis maximal 15 mm, vorzugsweise bis maximal 10 mm, einstellbar ist.

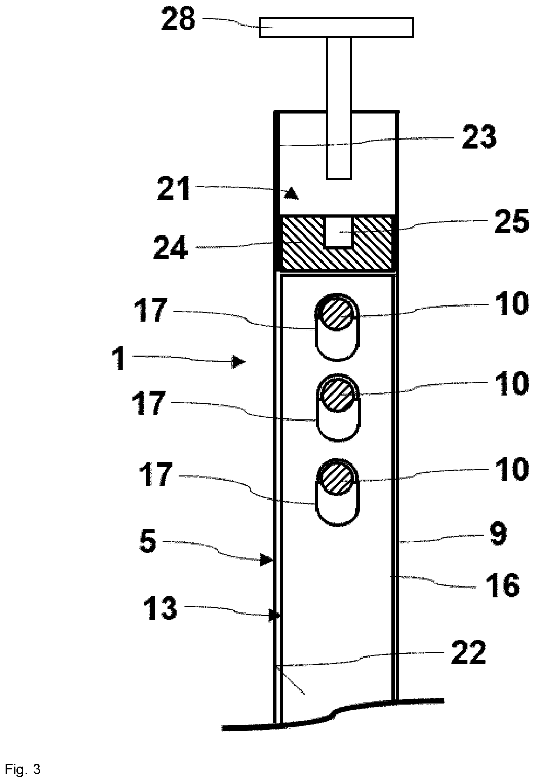

Zudem ist die Begrenzungseinrichtung 21 derart ausgebildet und angeordnet, dass die in Fig. 1 gezeigte erste axiale Endstellung der axialen Beweglichkeit gegeben ist, wenn der Marknagel 18 seine maximale axiale Länge 20 aufweist und eine von der ersten axialen Endstellung abweichende zweite Endstellung der axialen Beweglichkeit mittels der Begrenzungseinrichtung 21 individuell festlegbar ist, wie es in den Fign. 3 bis 6 gezeigt ist. Die Begrenzungseinrichtung 21 ist ausschließlich mechanisch ausgebildet.

Ein radialer Abstand zwischen der Aufnahmehülse 5 und dem Nagelteil 13 kann beispielsweise derart dimensioniert sein, dass die Aufnahmehülse 5 frei auf dem Nagelteil 13 gleiten kann. Ein radialer Abstand zwischen dem zu behandelnden Röhrenknochen 4 und dem Marknagel 18 kann derart dimensioniert sein, dass der Marknagel 18 im Rahmen einer intramedullären Osteosynthese in den Markräumen 6 und 7 des Röhrenknochens 4 "eingeschoben" werden kann und dass bei der Dynamisierung beide Knochenteile 2 und 3 noch axial auf dem Marknagel 18 gleiten können.

Die Begrenzungseinrichtung 21 weist ein an einer Innenmantelfläche 22 der Aufnahmehülse 5 angeordnetes Innengewinde 23 und eine in das Innengewinde 23 eingeschraubte Madenschraube 24 auf. Die zweite Endstellung der axialen Beweglichkeit der Aufnahmehülse 5 und des Nagelteils 13 relativ zueinander ist mittels der Madenschraube 24 individuell festlegbar, wie es in den Fign. 3 bis 6 gezeigt ist.

Eine axiale Höhe des Innengewindes 23 entspricht dem Doppelten einer axialen Höhe der Madenschraube 24. Die axiale Höhe der Madenschraube 24 entspricht der maximalen Beweglichkeit der Aufnahmehülse 5 und des Nagelteils 13 relativ zueinander. Die Madenschraube 24 bildet einen axialen Anschlag für das Nagelteil 13 und begrenzt somit die Beweglichkeit des Nagelteils 13 in einer axialen Richtung relativ zu der Aufnahmehülse 5.

Die Madenschraube 24 weist an ihrer dem Nagelteil 13 abgewandten Seite eine Mitnehmerkontur 25 auf, mit der ein in Fig. 3 gezeigtes Werkzeug formschlüssig verbunden werden kann, um die Madenschraube 24 betätigen zu können. Die Mitnehmerkontur 25 ist als Inbuskontur ausgebildet.

Der Fixierabschnitt 14 ist über drei in einer axialen Reihe beabstandet zueinander angeordnete, in das zweite Knochenteil 3 eingeschraubte Verriegelungsschrauben 10 an dem zweiten Knochenteil 3 fixiert. Hierzu sind an dem Fixierabschnitt 14 drei quer zu einer Längserstreckung L des Nagelteils 13 verlaufende Bohrungen 15 ausgebildet, durch die jeweils eine der Verriegelungsschrauben 10 formschlüssig hindurchgeführt ist. Jede Bohrung 15 ist als Durchgangsbohrung mit kreisrunder Querschnittsfläche ausgebildet.

An einem in dem Hülsenabschnitt 9 befindlichen Nagelabschnitt 16 des Nagelteils 13 sind drei quer zu einer Längserstreckung L des Nagelteils 13 verlaufende, in einer axialen Reihe beabstandet zueinander angeordnete Durchbrechungen 17 ausgebildet, durch die jeweils eine der Verriegelungsschrauben 10 hindurchgeführt ist und die jeweils in Form eines sich axial erstreckenden Langlochs ausgebildet sind. Ein der in das Innengewinde 23 eingeschraubten Madenschraube 24 zugewandt angeordnetes Ende der jeweiligen Durchbrechung 17 definiert die erste axiale Endstellung der axialen Beweglichkeit der Aufnahmehülse 5 und des Nagelteils 13 relativ zueinander.

Je nach Anwendungsfall können bzw. kann die Aufnahmehülse 5 und/oder das Nagelteil 13 zumindest teilweise gekrümmt ausgebildet sein.

Ferner weist die Marknagelanordnung 1 eine teilweise in das Innengewinde 23 einschraubbare Verschlusskappe 26 auf, mit der ein auf einer dem Nagelteil 13 abgewandten Seite der in das Innengewinde 23 eingeschraubten Madenschraube 24 angeordnetes axiales Ende der Aufnahmehülse 5 verschließbar ist. An der Verschlusskappe 26 ist eine Mitnehmerkontur 27 ausgebildet, deren Formgebung der Formgebung der Mitnehmerkontur 25 der Madenschraube 24 entspricht.

Alternativ kann für die Verschlusskappe 26 ein nicht gezeigtes eigenes Innengewinde an der Innenmantelfläche 22 der Aufnahmehülse 5 angeordnet sein, das anders als das Innengewinde 23 ausgebildet ist. Beispielsweise kann das Innengewinde für die Verschlusskappe 26 weniger robust ausgebildet sein, da geringere Kräfte auf die Verschlusskappe 26 einwirken als auf die Madenschraube 24. Eine axiale Höhe des Innengewindes für die Verschlusskappe 26 kann beispielsweise 3 mm betragen.

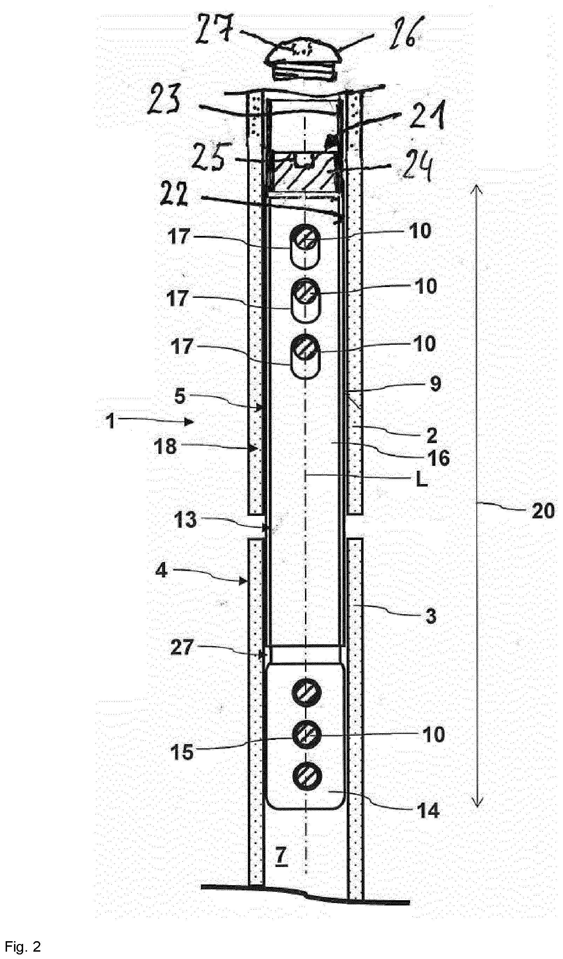

Fig. 2 zeigt einen weiteren schematischen Längsschnitt der in Fig. 1 gezeigten Osteosynthese-Marknagelanordnung 1, wobei diese Seitenansicht gegenüber der in Fig. 1 gezeigten Seitenansicht um 90° um die Längserstreckung L der Aufnahmehülse 5 bzw. des Nagelteils 13 gedreht ist.

In Fig. 2 ist insbesondere die Ausbildung der Durchbrechungen 17 an dem Nagelabschnitt 16 des Nagelteils 13 jeweils als axial verlaufendes Langloch zu sehen.

Fig. 3 zeigt einen schematischen Längsschnitt von Details der in Fig. 1 gezeigten Marknagelanordnung 1 in einem Ausgangszustand. Von der Marknagelanordnung 1 sind lediglich ein Abschnitt des Hülsenabschnitts 9 der Aufnahmehülse 5, ein Abschnitt des Nagelabschnitts 16 des Nagelteils 13 und die Madenschraube 24 gezeigt. Der Marknagel 18 kann in dem gezeigten Ausgangszustand in den frakturierten Röhrenknochen 4 eingebracht werden.

In dem Ausgangszustand ist die Madenschraube 24 derart in das Innengewinde 23 eingeschraubt, dass das Nagelteil 13 gegen die Verriegelungsschrauben 10 gedrängt wird und in diesem Zustand gehalten wird. Hierzu kann die Madenschraube 24 mittels eines als Inbusschlüssel ausgebildeten Werkzeugs 28 betätigt werden.

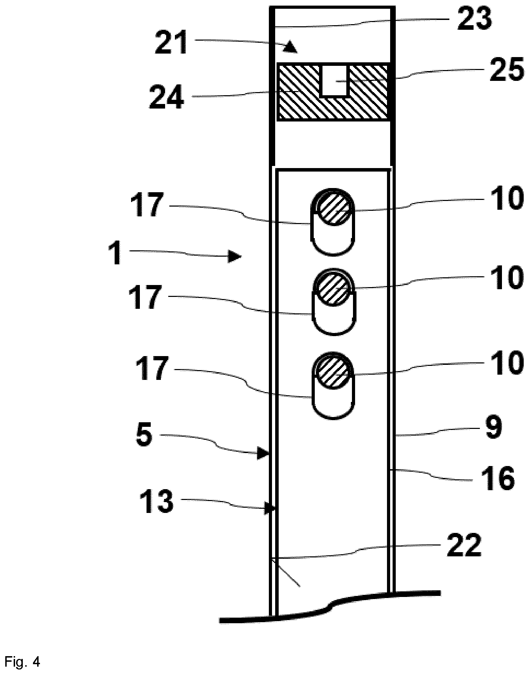

Fig. 4 zeigt einen schematischen Längsschnitt von Details der in Fig. 1 gezeigten Marknagelanordnung 1 in einem Dynamisierungszustand.

Der Dynamisierungszustand geht dadurch aus dem in Fig. 3 gezeigten Ausgangszustand hervor, dass die Madenschraube 24 mittels des in Fig. 3 gezeigten Werkzeugs von dem Nagelteil 13 weg verlagert worden ist. Insbesondere ist die Madenschraube 24 um 5 mm verlagert worden, wodurch eine Dynamisierung der Fraktur von 5 mm erfolgt. Die Indikation für eine Dynamisierung von 5 mm kann gestellt werden, wenn im Frakturspalt-Bereich eine Dehiszenz (Frakturspalt-Verbreiterung) von 5 mm besteht. Beide Frakturteile müssen wieder zum Kontakt miteinander gebracht werden, damit eine Knochen-Neubildung (Callus) im Frakturbereich entstehen bzw. sich bilden kann. Durch einen axialen Druck kann es zu Bewegungen der der Aufnahmehülse 5 und des Nagelteils 13 relativ zueinander kommen.

Durch die Verlagerung der Madenschraube 24 ist zwischen der Madenschraube 24 und dem Nagelteil 13 ein freier Raum mit 5 mm axialer Höhe entstanden, in dem sich das Nagelteil 13 zur Dynamisierung frei relativ zu der Aufnahmehülse 5 axial bewegen kann.

Mit dem in Fig. 4 gezeigten Dynamisierungszustand kann eine mittels der Madenschraube 24 auf 5 mm begrenzte und damit kontrollierte Dynamisierung der Fraktur in Form einer gegenseitigen Annäherung bis zur Kontaktaufnahme beider Knochenteile 2 und 3 erfolgen. Zudem kann eine unilaterale distale Dynamisierung erfolgen, bei der nur der untere, beispielsweise knienahe bzw. distale, Knochenteil 3 bewegt wird. Des Weiteren kann eine unilaterale proximale Dynamisierung erfolgen, bei der nur der obere, beispielsweise hüftnahe bzw. proximale, Knochenteil 2 bewegt wird. Darüber hinaus kann eine bilaterale distale und proximale Dynamisierung erfolgen, bei der beide Knochenteile 2 und 3 bewegt werden.

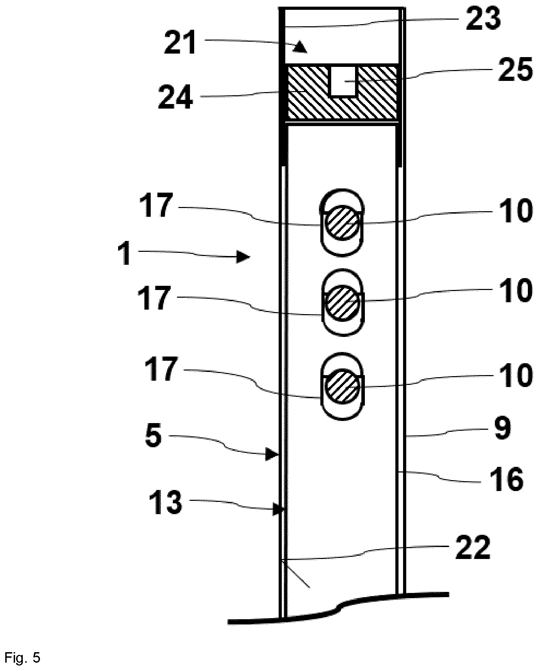

Fig. 5 zeigt einen schematischen Längsschnitt von Details der in Fig. 1 gezeigten Marknagelanordnung 1 in einem weiteren Dynamisierungszustand. Fig. 5 zeigt insbesondere ein Beispiel für eine unilaterale distale Dynamisierung der Fraktur. Hierbei bewegt sich das Nagelteil 13 um 5 mm in Richtung der Madenschraube 24 und somit das Knochenteil 3 um 5 mm in Richtung des Knochenteils 2. Dadurch wird eine Frakturspaltbreite von 5 mm ausgeglichen, respektive aufgehoben. Beide Knochenteile 2 und 3 stehen damit im Kontakt zueinander. Dieser Kontakt ist für eine positive Prognose der Frakturheilung ausschlaggebend. Eine weitere Bewegung des Nagelteils 13 relativ zu der Aufnahmehülse 5 erfolgt aufgrund des Widerstands der Madenschraube 24 nicht. Die Verriegelungsschrauben 10 befinden sich etwa mittig in den Durchbrechungen 11. Die Madenschraube 24 begrenzt somit die axiale Beweglichkeit des Nagelteils 13 relativ zu der Aufnahmehülse 5. In Fig. 5 ist die von der in den Fign. 1 bis 3 gezeigten ersten axialen Endstellung abweichende zweite Endstellung der axialen Beweglichkeit der Aufnahmehülse 5 und des Nagelteil 13 relativ zueinander gezeigt, die mittels der Begrenzungseinrichtung 21 bzw. der Madenschraube 24 individuell festgelegt ist.

Der Knochenteil 3 ist mit dem Nagelteil 13 fest verschraubt und wird deswegen immer mit dem Nagelteil 13 mitbewegt. Die Durchbrechungen 11 können beispielsweise als Langloch oder elliptisch ausgebildet sein und beispielsweise eine axiale Länge von 15 mm aufweisen. Ein Durchmesser eines Schaftes der jeweiligen Verriegelungsschraube 10 kann 5 mm betragen. Dann kann sich das Nagelteil 13 bei einer Verlagerung der Madenschraube um 10 mm um weitere 10 mm nach oben bewegen.

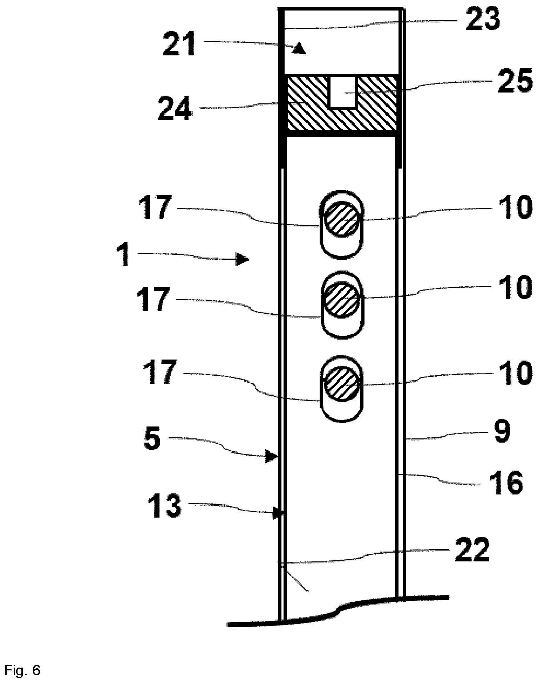

Fig. 6 zeigt einen schematischen Längsschnitt von Details der in Fig. 1 gezeigten Marknagelanordnung 1 in einem weiteren Dynamisierungszustand. Insbesondere zeigt Fig. 6 ein Beispiel für eine unilaterale, proximale Dynamisierung der Fraktur. Hierbei ist die Madenschraube 24 entsprechend Fig. 3 axial verlagert worden. Hierdurch ist zunächst zwischen der Madenschraube 24 und dem Nagelteil 13 eine Distanz von 5 mm geschaffen worden. Durch einen axialen Druck auf den frakturierten Röhrenknochen 4 hat sich das Nagelteil 13 um 5 mm in Richtung der Madenschraube 24 verlagert, so dass das Nagelteil 13 an der Madenschraube 24 anschlägt.

Wenn das Nagelteil 13 nicht nach oben bewegt wird, sondern nur die Aufnahmehülse 5 nach unten bewegt wird, kann in diesem Fall eine unilaterale proximale Dynamisierung der Fraktur erfolgen. Der proximale Knochenteil 2 ist fest mit der Aufnahmehülse 5 verschraubt und wird mit der Aufnahmehülse 5 ebenfalls um 5 mm nach unten, beispielsweise zum Knie hin, bewegt. Dadurch kann eine Frakturspaltbreite von 5 mm ausgeglichen werden. Danach stehen beide Knochenteile 2 und 3 in Kontakt miteinander. Dieser Kontakt ist für eine positive Prognose der Frakturheilung wichtig. Die Verriegelungsschrauben 10 gleiten hierbei in den Durchbrechungen 11 um 5 mm nach unten.

Bei einer bilateralen, distalen und proximalen Dynamisierung werden beide Knochenteile 2 und 3 anteilmäßig bewegt. Die Gesamtlänge der Dynamisierung von 5 mm wird dabei auf eine axiale Bewegung der Aufnahmehülse 5 und einer axialen Bewegung des Nagelteils 13 aufgeteilt.

### Bezugszeichenliste

- 1: Osteosynthese-Marknagelanordnung
- 2: erstes Knochenteil
- 3: zweites Knochenteil
- 4: Röhrenknochen
- 5: erstes Nagelelement in Form einer Aufnahmehülse
- 6: Markraum von 2
- 7: Markraum von 3
- 9: Hülsenabschnitt von 5
- 10: Verriegelungsschraube
- 11: Durchbrechung an 9
- 12: Durchbrechungsöffnung von 11
- 13: zweites Nagelelement in Form eines Nagels
- 14: Fixierabschnitt
- 15: Bohrung an 14
- 16: Nagelabschnitt
- 17: Durchbrechung an 16
- 18: Marknagel
- 20: Länge von 18
- 21: Begrenzungseinrichtung
- 22: Innenmantelfläche von 5
- 23: Innengewinde an 22
- 24: Madenschraube
- 25: Mitnehmerkontur an 24
- 26: Verschlusskappe
- 27: Mitnehmerkontur an 26
- 28: Werkzeug
- L: Längserstreckung von 5, 13

## Patentansprüche

1. Osteosynthese-Marknagelanordnung (1) zur Osteosynthese von Knochenteilen (2, 3) eines frakturierten Röhrenknochens (4), aufweisend wenigstens einen Marknagel (18) mit variierbarer axialer Länge (20), **dadurch gekennzeichnet, dass** der Marknagel (18) umfasst
- wenigstens ein erstes Nagelelement (5) in Form einer Aufnahmehülse, die zumindest teilweise in einen Markraum (6) eines ersten Knochenteils (2) des frakturierten Röhrenknochens (4) einfügbar ist, wobei ein Hülsenabschnitt (9) des ersten Nagelelements (5) über wenigstens eine Verriegelungsschraube (10) an dem ersten Knochenteil (2) fixierbar ist,
- wenigstens ein teilweise axial in das erste Nagelelement (5) eingreifendes zweites Nagelelement (13) in Form eines Nagels, das einen außerhalb des ersten Nagelelements (5) angeordneten, in einen Markraum (7) des zweiten Knochenteils (3) einfügbaren und an dem zweiten Knochenteil (3) fixierbaren Fixierabschnitt (14) aufweist, über das das Nagelelement (13) mit wenigstens einer Verriegelungsschraube (10) an dem zweiten Knochenteil (3) fixierbar ist, und
- wenigstens eine Begrenzungseinrichtung (21) zum Begrenzen einer axialen Beweglichkeit des zweiten Nagelelements (13) relativ zu dem ersten Nagelelement (5) in Richtung eines dem Fixierabschnitt (14) abgewandten Ende des ersten Nagelelements (5),
wobei an einem in dem Hülsenabschnitt (9) befindlichen Nagelabschnitt (16) des zweiten Nagelelements (13) wenigstens eine quer zu einer Längserstreckung (L) des zweiten Nagelelements (13) verlaufende Durchbrechung (17) in Form eines sich axial erstreckenden Langlochs ausgebildet ist, durch die die Verriegelungsschraube (10) hindurchgeführt ist.

2. Osteosynthese-Marknagelanordnung (1) nach Anspruch 1, **dadurch gekennzeichnet, dass** die Begrenzungseinrichtung (21) derart ausgebildet und angeordnet ist, dass die axiale Beweglichkeit individuell in einem Bereich von 0 mm bis maximal 15 mm, vorzugsweise bis maximal 10 mm, einstellbar ist und dass eine erste axiale Endstellung der axialen Beweglichkeit gegeben ist, wenn der Marknagel (18) seine maximale axiale Länge (20) aufweist und eine von der ersten axialen Endstellung abweichende zweite Endstellung der axialen Beweglichkeit mittels der Begrenzungseinrichtung (21) individuell festlegbar ist.

3. Osteosynthese-Marknagelanordnung (1) nach einem der vorherigen Ansprüche, **dadurch gekennzeichnet, dass** die Begrenzungseinrichtung (21) wenigstens ein an einer Innenmantelfläche des ersten Nagelelements (5) angeordnetes Innengewinde (23) und wenigstens eine in das Innengewinde (23) einschraubbare Madenschraube (24) aufweist.

4. Osteosynthese-Marknagelanordnung (1) nach Anspruch 3, **dadurch gekennzeichnet, dass** die in das Innengewinde (23) eingeschraubte Madenschraube (24) zugewandt angeordnetes Ende der Durchbrechung (17) die erste axiale Endstellung der axialen Beweglichkeit definiert.

5. Osteosynthese-Marknagelanordnung (1) nach Anspruch 3 oder 4, **gekennzeichnet durch** wenigstens eine teilweise in das Innengewinde (23) einschraubbare Verschlusskappe (26), mit der ein auf einer dem zweiten Nagelelement (13) abgewandten Seite der in das Innengewinde (23) eingeschraubten Madenschraube (24) angeordnetes axiales Ende des ersten Nagelelements (5) verschließbar ist.

6. Osteosynthese-Marknagelanordnung (1) nach Anspruch 4 oder 5, **dadurch gekennzeichnet, dass** die zweite Endstellung der axialen Beweglichkeit mittels der Madenschraube (24) individuell festlegbar ist.

7. Osteosynthese-Marknagelanordnung (1) nach einem der vorherigen Ansprüche, **dadurch gekennzeichnet, dass** das erste Nagelelement (5) teilweise in einen Markraum (7) des zweiten Knochenteils (3) des frakturierten Röhrenknochens (4) einfügbar ist.

8. Osteosynthese-Marknagelanordnung (1) nach einem der vorherigen Ansprüche, **dadurch gekennzeichnet, dass** der Hülsenabschnitt (9) über wenigstens drei in einer axialen Reihe beabstandet zueinander angeordnete Verriegelungsschrauben (10) an dem ersten Knochenteil (2) fixierbar ist, wobei an dem Nagelabschnitt (16) drei quer zu der Längserstreckung (L) des zweiten Nagelelements (13) verlaufende, in einer axialen Reihe beabstandet zueinander angeordnete Durchbrechungen (17) in Form von sich axial erstreckenden Langlöchern ausgebildet sind, durch die die Verriegelungsschrauben (10) hindurchgeführt sind.

9. Osteosynthese-Marknagelanordnung (1) nach einem der vorherigen Ansprüche, **dadurch gekennzeichnet, dass** ein Außendurchmesser des Fixierabschnitts (14) einem Außendurchmesser des ersten Nagelelements (5) entspricht.

10. Osteosynthese-Marknagelanordnung (1) nach einem der vorherigen Ansprüche, **dadurch gekennzeichnet, dass** der Fixierabschnitt (14) über wenigstens zwei in einer axialen Reihe beabstandet zueinander angeordnete Verriegelungsschrauben (10) an dem zweiten Knochenteil (3) fixierbar ist, wobei an dem Fixierabschnitt (14) wenigstens zwei quer zu einer Längserstreckung (L) des zweiten Nagelelements (13) verlaufende Bohrungen (15) ausgebildet sind, durch die jeweils eine der Verriegelungsschrauben (10) formschlüssig hindurchführbar ist.

11. Osteosynthese-Marknagelanordnung (1) nach einem der vorherigen Ansprüche, **dadurch gekennzeichnet, dass** das erste Nagelelement (5) und/oder das zweite Nagelelement (13) zumindest teilweise gekrümmt ausgebildet sind bzw. ist.

12. Osteosynthese-Marknagelanordnung (1) nach einem der vorherigen Ansprüche, **dadurch gekennzeichnet, dass** die Begrenzungseinrichtung (21) wenigstens ein elektromotorisches Stellmittel aufweist, mit dem eine Position der in das Innengewinde eingeschraubten Madenschraube (24) relativ zu dem ersten Nagelelement (5) variierbar ist.

13. Osteosynthese-Marknagelanordnung (1) nach einem der vorherigen Ansprüche, **dadurch gekennzeichnet, dass** die Begrenzungseinrichtung (21) ausschließlich mechanisch ausgebildet ist.

14. Osteosynthese-Marknagelanordnung (1) nach einem der vorherigen Ansprüche, **dadurch gekennzeichnet, dass** wenigstens eine weitere Begrenzungseinrichtung zum verliersicheren Begrenzen einer axialen Beweglichkeit des zweiten Nagelelements (13) relativ zu dem ersten Nagelelement (5) in Richtung eines dem Fixierabschnitt (14) des abgewandten Endes des ersten Nagelelements (5) weg.
